# EUROPÄISCHE PATENTANMELDUNG

(11) **EP 1 970 041 A1**
(43) Veröffentlichungstag der Anmeldung: **17.09.2008**
(21) Anmeldenummer: 07005445.7
(22) Anmeldetag: 16.03.2007
(51) Int. Cl.: A61K 6/00, A61K 6/083

(54) **Kit zum Befestigen eines dentalen Restaurationsmaterials an einer Zahnsubstanz**

(71) Anmelder: Ernst Mühlbauer GmbH & Co.KG, 25870 Norderfriedrichskoog (DE)
(72) Erfinder: Ziegler, Silke, 22529 Hamburg (DE)
(74) Vertreter: Glawe, Delfs, Moll

(57) **Zusammenfassung**

Gegenstand der Erfindung ist ein Kit zum Befestigen eines dentalen Restaurationsmaterials an einer Zahnsubstanz, der folgende Komponenten enthält:
a. eine Elektronendonorverbindung;
b. eine erste Klebstoffkomponente, die ein Oxidationsmittel als Bestandteil eines Polymerisationsinitiatorsystems und eine polymerisierbare Säure enthält;
c. eine zweite Klebstoffkomponente, die ein Reduktionsmittel als Bestandteil eines Polymerisationsinitiatorsystems und ein polymerisierbares Monomer, Oligomer oder Präpolymer enthält, das keine Säuregruppen enthält.

## Beschreibung

Die Erfindung betrifft ein Kit zum Befestigen eines dentalen Restaurationsmaterials an einer Zahnsubstanz.

In der Zahnheilkunde ist es bekannt, dentale Restaurationsmaterialien, beispielsweise Kunststoffmaterialien wie Komposite, mittels Dentalklebstoffen an einer Zahnsubstanz, insbesondere Dentin, zu befestigen. Häufig wird dabei das Dentin vor dem Auftragen des Dentalklebstoffes mit einer Säure angeätzt.

Aus EP 0 767 652 B1 ist ein eingangs genanntes Verfahren bekannt, bei dem vor dem Auftragen des Dentalklebers zunächst mit einer Säure geätzt und dann ein Primer aufgetragen wird.

Der Erfindung liegt die Aufgabe zugrunde, einen Kit der eingangs genannten Art zu schaffen, der bei einfacher Anwendung eine gute Haftvermittlung zwischen Zahnsubstanz und dentalem Restaurationsmaterial bewirkt.

Ein erfindungsgemäßer Kit weist folgende Komponenten auf:
a. eine Elektronendonorverbindung;
b. eine erste Klebstoffkomponente, die ein Oxidationsmittel als Bestandteil eines Polymerisationsinitiatorsystems und eine polymerisierbare Säure enthält;
c. eine zweite Klebstoffkomponente, die ein Reduktionsmittel als Bestandteil eines Polymerisationsinitiatorsystems und ein polymerisierbares Monomer, Oligomer oder Präpolymer enthält, das keine Säuregruppen enthält.

Der erfindungsgemäße Kit enthält somit wenigstens drei Komponenten. Die erste Komponente enthält eine Elektronendonorverbindung. Die Elektronendonorverbindung hat ein E_{OX}>0 V und vorzugsweise etwa 0,5 bis 1 V gegen eine gesättigte Kalomelelektrode (SCE) als Referenz. Bevorzugte E-lektronendonorverbindungen sind geeignete aromatische organische Säuren oder Salze dieser Säuren wie beispielsweise eine aromatische Sulfinsäure oder deren Salz oder Barbitursäure oder ein Barbitursäurederivat. Geeignet Sulfinsäuresalze sind beispielsweise die Alkalimetallsalze, Erdalkalimetallsalze, Aminsalze oder Ammoniumsalze. Die Alkalimetallsalze können insbesondere Lithium-, Natrium- oder Kaliumsalze sein. Unter den Erdalkalimetallsalzen sind Magnesium-, Calcium-, Strontium- und Bariumsalze bevorzugt. Die Aminsalze können primäre Aminsalze sein, die beispielsweise folgende Gruppen aufweisen: Methylamin, Ethylamin, Propylamin, Butylamin, Anilin, Toluidin, Phenylendiamin oder Xylylendiamin. Sekundäre Aminsalze können folgende Gruppen aufweisen: Dimethylamin, Diethylamin, Dipropylamin, Dibutylamin, Piperidin, N-Methylanilin, N-Ethylanilin, Diphenylamin oder N-Methyltoluidin. Tertiäre Amine können folgende Gruppen aufweisen: Trimethylamin, Triethylamin, Pyridin, N,N-Dimethylanilin, N,N-di(beta-Hydroxyethyl)anilin, N,N-Diethylamin, N,N-Dimethyltoluidin, N,N-Diethyltoluidin oder N,N-(beta-Hydroxyethyl)toluidin. Als Ammoniumsalze können beispielsweise Tetramethylammoniumsalze, Tetraethylammoniumsalze, Tetrapropylammoniumsalze oder Trimethylbenzylammoniumsalze verwendet werden.

Geeignete organische Sulfinsäuren sind Alkansulfinsäuren (beispielsweise Ethan-, Propan-, Hexan-, Octan-, Decan-, und Dodecansulfinsäure); alicyclische Sulfinsäuren wie Cyclohexan- und Cyclooctansulfinsäure; sowie aromatische Sulfinsäuren wie Benzolsulfinsäure, o-Toluolsulfinsäure, p-Toluolsulfinsäure, Ethylbenzolsulfinsäure, Decylbenzolsulfinsäure, Dodecylbenzolsulfinsäure, Chlorbenzolsulfinsäure und Naphthalensulfinsäure.

Geeignete organische Sulfinsäuresalze sind beispielsweise Benzolsulfinsäuresalze mit folgenden Gegenionen: Natrium, Kalium, Magnesium, Calcium, Strontium, Barium, Butylamin, Anilin, Toluidin, Phenylendiamin, Diethylamin, Diphenylamin, Triethylamin, Ammonium, Tetremethylammonium, und Trimethylbenzylammonium. Geeignete Gegenionen für o-Toluolsulfinsäuresalze sind beispielsweise Lithium, Natrium, Kalium, Calcium, Cyclohexylamin, Anilin, Ammonium, und Tetraethylammonium. Geeignete Gegenionen für p-Toluolsulfinsäuresalze sind beispielsweise Lithium, Natrium, Kalium, Calcium, Barium, Ethylamin, Toluidin, N-Methylanilin, Pyridin, Ammonium und Tetramethylammonium. Geeignete Gegenionen für Beta-Naphthalensulfinsäuresalze sind beispielsweise Natrium, Strontium, Triethylamin, N-Methyltoluidin, Ammonium, Trimethylbenzylammonium und dergleichen.

Geeignete Barbitursäuren sind beispielsweise 1,3,5-Trimethylbarbitursäure, 1,3,5-Triethylbarbitursäure, 1,3-Dimethyl-5-Ethylbarbitursäure, 1,5-Dimethylbarbitursäure, 1-Methyl-5-Ethylbarbitursäure, 1-Methyl-5-Propylbarbitursäure, 5-Ethylbarbitursäure, 5-Propylbarbitursäure, 5-Butylbarbitursäure, 5-Methyl-1-Butylbarbitursäure, 1-Benzyl-5-Phenylbarbitursäure, 1-Cyclohexyl-5-Ethylbarbitursäure oder Alkalimetallsalze der vorgenannten Säuren.

Besonders bevorzugte aromatische Sulfinatsalze sind Natriumbenzolsulfinat und Natriumtoluolsulfinat.

Die erste und zweite Klebstoffkomponente enthalten jeweils ein Oxidationsmittel bzw. Reduktionsmittel als Bestandteil eines Polymerisationsinitiatorsystems. Ein solches RedoxKatalysatorsystem erzeugt durch Zusammenwirken von Reduktions- und Oxidationsmittel freie Radikale, die in der Lage sind, die Polymerisation ethylenisch ungesättigter polymerisierbarer Bestandteile zu initiieren.

Geeignete Oxidationsmittel sind beispielsweise Persulfate und bevorzugt organische Peroxide. Beispielhaft genannt seien Diacetylperoxid, Dipropylperoxid, Dibutylperoxid, Dicaprylperoxid, Dilaurylperoxid, Dibenzoylperoxid (BPO), p,p'-Dichlorbenzoylperoxid, p,p'-Dimethoxybenzoylperoxid, p,p'-Dimethylbenzoylperoxid und p,p'-Dinitrodibenzoylperoxid.

Als Reduktionsmittel des Polymerisationsinitiatorsystems können insbesondere aliphatische, alicyclische oder aromatische Amine verwendet werden. Es können primäre, sekundäre oder tertiäre Amine verwendet werden. Bevorzugt sind aromatische Amine, insbesondere tertiäre aromatische Amine. Unter den tertiären aromatischen Aminen sind N,N-bis-(2-Hydroxyethyl)-p-Toluidin, N,N-Dimethyl-p-tert-butylanilin, N,N-Diethylaminobezoesäure (DEABA) und 4-(Dimethylamino)Phenethylalkohol besonders bevorzugt

Die erste Klebstoffkomponente enthält eine polymerisierbare Säure. "Polymerisierbar" bedeutet in diesem Zusammenhang, dass diese Säure ethylenisch ungesättigte Doppelbindungen enthält, über die, ausgelöst durch das Polymerisationsinitiatorsystem, eine Polymerisation bzw. Vernetzung stattfinden kann. Die Säurefunktionen der polymerisierbaren Säure dienen insbesondere zur festen Verbindung mit der Zahnsubstanz, insbesondere mit Dentin oder Zahnschmelz. Geeignete polymerisierbare Säuren sind beispielsweise polymerisierbare Homopolymere oder Copolymere einer α, β-ungesättigten Karbonsäure. Beispielhaft genannt seien (Meth)Acrylpolyacrylsäuren, oder Copolymere aus der genannten Säure einerseits und beispielsweise Maleinsäure und/oder Itaconsäure andererseits. Bevorzugt weist die polymerisierbare Säure eine mittlere Molmasse von 2.000 bis 100.000, vorzugsweise 5.000 bis 60.000, weiter vorzugsweise 10.000 bis 50.000, weiter vorzugsweise 20.000 bis 40.000 auf.

Die zweite Klebstoffkomponente weist ein polymerisierbares Monomer, Oligomer oder Präpolymer auf, das keine Säuregruppen enthält. Es kann sich dabei um ein Monomer mit mindestens einer ethylenisch ungesättigten Gruppe handeln, beispielsweise ein Acrylat, Methacrylat oder ein Monomer, Oligomer oder Präpolymer mit einer Vinylgruppe. Besonders bevorzugt sind Monomere mit Acrylat- oder Methacrylatgruppen. Beispielhaft genannt seien Hydroxyethyl(Meth)Acrylat, Hydroxypropyl(Meth)Acrylat, Hydroxybutyl(Meth)Acrylat, Glycerindi(Meth)Acrylat, Glycerinmono(Meth)Acrylat, Methyl(Meth)Acrylat, Ethyl(Meth)Acrylat, Propyl(Meth)Acrylat, Butyl(Meth)Acrylat, Hexyl(Meth)Acrylat, Octyl(Meth)Acrylat, Lauryl(Meth)Acrylat, Decyl(Meth)Acrylat, Tridecyl(Meth)Acrylat, 2-Ethoxyethyl(Meth)Acrylat, 2'-Ethoxy-2-Ethoxyethyl(Meth)Acrylat, Ethylenglycoldi(Meth)Acrylat, Diethylenglycoldi(Meth)Acrylat, Triethylenglycoldi(Meth)Acrylat (TEGDMA), Tetraethylenglycoldi(Meth)Acrylat, Polyethylenglycolmono(Meth)Acrylat, Polyethylenglycoldi(Meth)Acrylat, Polypropylenglycolmono(Meth)Acrylat, Polypropylenglycoldi(Meth)Acrylat, Polytetramethylenglycolmono(Meth)Acrylat, Polytetrametylenglycoldi(Meth)Acrylat, Hexandioldi(Meth)Acrylat, Trimethyloylpropantri(Meth)Acrylat, UDMA (Reaktionsprodukt von 2-Hydroxyethylmethacrylat (HEMA) mit 2,4,4-Trimethylhexandiisocyanat), 2,2-bis[4-(2-Hydroxy-3-Methacryloylpropoxy)-phenyl]-Propan (Bis-GMA), ethoxyliertes Bisphenol-A-Dimethacrylat und dergleichen. Hydroxyethylmethacrylat (HEMA) ist als polymerisierbares Monomer ohne Säuregruppen besonders bevorzugt.

Die Anwendung des erfindungsgemäßen Kits geschieht wie folgt:

Die zu behandelnde Zahnsubstanz wird zunächst in herkömmlicher Weise vorbereitet. Es kann zunächst eine Präparation mit Werkzeugen wie beispielsweise einem Bohrer erfolgen. Anschließend wird die Oberfläche gespült und getrocknet.

Im nächsten Schritt wird die zu behandelnde Dentaloberfläche mit Säure geätzt. Geeignete Säuren können anorganische oder organische Säuren sein, sie weisen bevorzugt einen pKa in Wasser auf, der kleiner als der oder gleich dem pKa-Wert von Phenol ist. Bevorzugte pKa-Werte liegen zwischen -20 und +10, weiter vorzugsweise zwischen -10 und +5. Geeignete anorganische Säuren sind beispielsweise Salzsäure, Salpetersäure und Bromwasserstoffsäure. Geeignete organische Säuren sind beispielsweise Ameisensäure, Trifluoressigsäure, Trichloressigsäure, Dichloressigsäure, Chloressigsäure, Bromessigsäure, Di- und Tribromessigsäure, Essigsäure, Alpha-Chlorpropionsäure, Propionsäure, Maleinsäure, Fumarsäure, Acrylsäure, Methacrylsäure, Trihydroxybenzoesäure, Benzoesäure, Toluolsulfonsäure, Phenol und geeignete Phenolderivate, Phosphorsäure, geeignete Phosphor(III)Säureester und dergleichen. Bevorzugt sind Säuren, die beim Ätzvorgang keine unlöslichen Calciumsalze auf der Oberfläche der Zahnsubstanz erzeugen, die die Haftung des darauf aufzubringenden dentalen Restaurationsmaterials beinträchtigen könnten. Entstehen solche Salze, können diese ggf. nach dem Ätzschritt abgespült werden.

Nach dem Ätzen wird die Elektronendonorverbindung aufgetragen. Im Rahmen der erfindungsgemäßen Kits liegt diese Elektronendonorverbindung bevorzugt in einem physiologisch akzeptablen Lösungsmittel vor. Geeignete Lösungsmittel sind beispielsweise Wasser, Aceton, Alkylalkohole wie Ethanol oder Propanol oder dergleichen. Bevorzugt ist es, dass das physiologisch akzeptable Lösungsmittel bei Körpertemperatur leicht flüchtig ist, wie beispielsweise Alkohol. "Physiologisch akzeptabel" bedeutet im Rahmen der Erfindung, dass das Lösungsmittel bei der üblichen Applikation nicht oder nur unwesentlich nachteilig auf den Organismus wirkt.

Die Elektronendonorverbindung ist bevorzugt in einem Anteil von 0,1 bis 20 Gew.-%, weiter vorzugsweise 0,5 bis 15 Gew.-%, weiter vorzugsweise 1 bis 10 Gew.-% in dem Lösungsmittel gelöst. Sie kann durch geeignete Applikatoren (Schwamm, Bürste oder dergleichen) aufgetragen werden, anschließend wird das Lösungsmittel bevorzugt verdunsten gelassen.

Im nächsten Schritt erfolgt das Auftragen einer Mischung der ersten und zweiten Klebstoffkomponente. Diese Mischung wird bevorzugt erst unmittelbar vor der Applikation auf die behandelte Zahnsubstanz hergestellt. Das Herstellen der Mischung kann manuell beispielsweise durch Verrühren mit einem Pinsel geschehen. Bevorzugt ist das Herstellen der Mischung unter Verwendung eines Applikators, wie er unten näher beschrieben werden wird.

Diese Mischung wird als Dentalklebstoff auf die Zahnsubstanz aufgetragen. Das Redoxkatalysatorsystem initiiert eine Polymerisation und letztendlich Aushärtung des Klebstoffs. Im Rahmen der Erfindung ist es möglich, dass zusätzlich eine Lichthärtung vorgenommen wird, wenn der ersten und/oder zweiten Klebstoffkomponente zusätzlich Fotopolymerisationsinitiatoren zugesetzt werden. Geeignete durch sichtbares Licht induzierte Fotoinitiatoren sind dem Fachmann geläufig und umfassen beispielsweise Alpha-Diketone wie Campherchinon, Diaryliodoniumsalze und dergleichen.

Das dentale Restaurationsmaterial wird auf die mit dem Dentalklebstoff beschichtete Zahnsubstanz aufgetragen, bevorzugt, bevor der Dentalklebstoff vollständig ausgehärtet ist. Das dentale Restaurationsmaterial wird bevorzugt unmittelbar nach Auftragung des Dentalklebstoffs auf die mit dem Dentalklebstoff beschichtete Zahnsubstanz aufgetragen. Geeignete dentale Restaurationsmaterialien sind insbesondere selbsthärtende Füllungskunststoffe wie beispielsweise Dentalkomposite.

Die Erfindung hat erkannt, dass sich überraschenderweise mit einem erfindungsgemäßen Kit auch ohne den im Stand der Technik für erforderlich gehalten separaten Primerschritt (zwischen Ätzschritt und Auftragen des Dentalklebstoffes) hohe Haftwerte erreichen lassen. Die Erfindung kombiniert somit eine vereinfachte Handhabung mit mindestens gleich guten, in der Regel sogar besseren Haftwerten als der Stand der Technik (beispielsweise gemäß EP 0 767 652 B1).

Im Rahmen der Erfindung ist es besonders bevorzugt, wenn die erste und/oder zweite Klebstoffkomponente zusätzlich einen Filmbildner aufweist. Mit dem Begriff Filmbildner wird jede Substanz bezeichnet, die auf der geätzten Zahnsubstanz einen im wesentlichen die Oberfläche lückenlos benetzenden Film bilden kann und die härtbar, insbesondere polymerisierbar ist. Der Filmbildner ist bevorzugt wenigstens so gut in Wasser löslich, dass eine Lösung von 5 Gew.-% dieses Filmbildners in Wasser hergestellt werden kann. Bevorzugt ist er mit Wasser vollständig mischbar. Filmbildner enthalten bevorzugt hydrophile Gruppen wie beispielsweise Hydroxylgruppen, Carboxylgruppen, Sulfonsäuregruppen, oder dergleichen. Bevorzugt enthält der Filmbildner polymerisierbare Gruppen wie z.B. Vinylgruppen, besonders bevorzugt Acrylat- oder Methacrylatgruppen.

Der Filmbildner kann ganz oder teilweise stofflich identisch sein mit dem polymerisierbaren Monomer der zweiten Klebstoffkomponente, das keine Säuregruppen enthält. Anders ausgedrückt kann eine bestimmte oder eine Mischung aus mehreren Substanzen gleichzeitig Filmbildner und polymersierbares Monomer der zweiten Klebstoffkomponente sein.

Zu den bevorzugten Filmbildnern gehören 2-Hydroxyethylacrylat, 2-Hydroxyethylmethacrylat ("HEMA"), 2- und 3-Hydroxypropylacrylat und -methacrylat, 1,3- und 2,3-Dihydroxypropylacrylat und -methacrylat, 2-Hydroxypropyl-1,3-diacrylat und -dimethacrylat, 3-Hydroxypropyl-1,2-diacrylat und -dimethylacrylat, Pentaerythritoldiacrylat und -dimethacrylat, Acrylsäure, Methacrylsäure, 2-Trimethylammoniumethylmethacrylchlorid, 2-Acrylamido-2-methylpropansulfonsäure, Acrylamid, Methacrylamid, 2-Hydroxyethylacrylamid und -methacrylamid, N,N-Bis(2-hydroxytethyl)acrylamid und -methacrylamid, N-Alkyl-N-hydroxyethylacrylamid und -methacrylamid, 2- und 3-Hydroxypropylacrylamid und -methacrylamid, Methacrylamidopropyltrimethylammoniumchlorid, Polyethylenglykol(400)-diacrylat und -dimethacrylat und Gemische derselben.

Besonders bevorzugte Filmbildner sind: 2-Hydroxymethylmethacrylat; 2- und 3-Hydroxypropylacrylat; 2- und 3-Hydroxypropylmethacrylat; 1,3- und 2,3-Dihydroxypropylacrylat; und 1,3- und 2,3-Dihydroxypropylmethacrylat.

Der Anteil des Filmbildners an der Gesamtmasse der ersten und zweiten Klebstoffkomponente, wenn diese im vorgesehenen Mischungsverhältnis angemischt sind, beträgt vorzugsweise mindestens 30 Gew.-%, weiter vorzugsweise mindestens 40 Gew.-%, weiter vorzugsweise mindestens 50 Gew.-%. Bevorzugte Obergrenzen sind 70 Gew.-%, besonders bevorzugt 60 Gew.-%. Im Rahmen bevorzugter Ausführungsformen der Erfindung können die genannten Obergrenzen mit den genannten Untergrenzen beliebig zu Bereichen kombiniert werden.

Bei einer Ausführungsform der Erfindung wird die erste und zweite Klebstoffkomponente des Kits gemeinsam in einem Applikator bereitgestellt, in dem sie getrennt gelagert sind. Der Applikator weist Einrichtungen zur Vermischung der ersten und zweiten Klebstoffkomponente und zur Entnahme der Mischung auf. Geeignete Applikatoren sind beispielsweise offenbart in WO 2007/017255 A2, die durch Bezugnahme darauf zum Gegenstand der Offenbarung der vorliegenden Anmeldung gemacht wird.

Der Applikator kann zusätzlich die Elektronendonorverbindung bereitstellen, die getrennt von den Klebstoffkomponenten gelagert und separat entnehmbar ist. Bei dieser Ausführungsform der Erfindung kombiniert der Applikator somit alle drei Komponenten des Kits in einem einzigen Applikator. Zwei der Komponenten (die erste und zweite Klebstoffkomponente) sind vor der Entnahme vermischbar, die dritte Komponente (die Elektronendonorverbindung) ist mittels des Applikators separat entnehmbar und applizierbar.

Im Rahmen der Erfindung kann der Kit zusätzlich eine Säurekomponente zum Ätzen der Zahnsubstanz enthalten. Es ist somit einerseits möglich, dass der Kit nur aus drei Komponenten besteht und bei der Applikation der Zahnarzt auf eine übliche Säure zum Ätzen der Zahnsubstanz zusätzlich zurückgreift. Im Rahmen der genannten Ausführungsformen der Erfindung kann die Säure jedoch auch als vierte Komponente des erfindungsgemäßen Kits bereitgestellt werden.

Als weitere Komponente des Kits kann auch das dentale Restaurationsmaterial wie beispielsweise ein dentaler Komposit bereitgestellt werden. Alternativ kann der Anwender des Kits auf übliche und im Handel erhältliche dentale Restaurationsmaterialien zusätzlich zu dem Kit zurückgreifen.

Der Kit kann zusätzlich eine Anleitung zur Applikation umfassen. Die Anleitung zur Applikation nennt bevorzugt folgende Schritte:
a. Ätzen der Zahnsubstanz mit einer Säure,
b. Auftragen der Elektronendonorverbindung auf die geätzte Zahnsubstanz,
c. Mischen der ersten und zweiten Klebstoffkomponente und Auftragen der Mischung auf die Zahnsubstanz,
d. Applizieren des dentalen Restaurationsmaterials auf die Zahnsubstanz.

Ein weiterer Gegenstand der Erfindung ist die Verwendung folgender Komponenten:
a. eine Elektronendonorverbindung;
b. eine erste Klebstoffkomponente, die ein Oxidationsmittel als Bestandteil eines Polymerisationsinitiatorsystems und eine polymerisierbare Säure enthält;
c. eine zweite Klebstoffkomponente, die ein Reduktionsmittel als Bestandteil eines Polymerisationsinitiatorsystems und ein polymerisierbares Monomer, Oligomer oder Präpolymer enthält, das keine Säuregruppen enthält; zur Herstellung eines Kits zum Befestigen eines dentalen Restaurationsmaterials an einer Zahnsubstanz, wobei die Komponenten des Kits hergerichtet sind zur Applikation umfassend folgende Schritte:
   - Ätzen der Zahnsubstanz mit einer Säure,
   - Auftragen der Elektronendonorverbindung auf die geätzte Zahnsubstanz,
   - Mischen der ersten und zweiten Klebstoffkomponente und Auftragen der Mischung auf die Zahnsubstanz,
   - Applizieren des dentalen Restaurationsmaterials auf die Zahnsubstanz.

Die vorstehend im Zusammenhang mit dem Kit erläuterten Vorteile und bevorzugten Ausführungsformen gelten sinngemäß auch für die hier genannte erfindungsgemäße Verwendung.

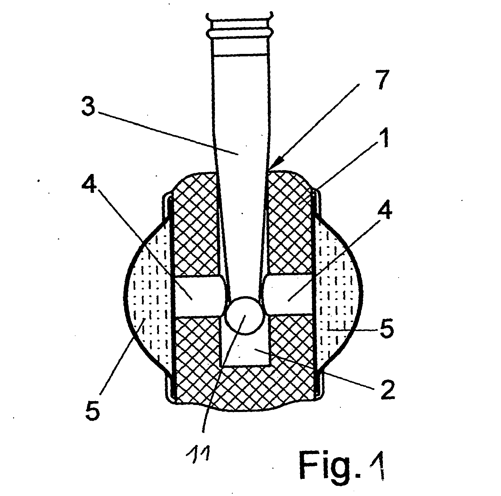

Die Erfindung wird nachfolgend anhand von Beispielen erläutert. Darin zeigen die Figuren 1 bis 5 Ausführungsformen der Erfindung, bei denen der Kit in einem Applikator bereitgestellt wird.

### Vergleichsbeispiel 1

Das in diesem Vergleichsbeispiel 1 verwendete Klebstoffsystem entspricht dem Beispiel 4 der EP 0 767 652 B1. Vier Komponenten folgender Zusammensetzung wurden hergestellt

| **Aktivator** | **Gew.-%** |
|---|---|
| Ethanol | 97 |
| Benzolsulfinsäue Na salz | 3 |
| | 100 |
| | |

| **Primer** | **Gew.-%** |
|---|---|
| methacrylierte Polyacrylsäure | 13,3 |
| Wasser | 46,9 |
| HEMA - Hydroxyethyldimethacrylat | 39,8 |
| | 100 |

| **Peroxid-Harz (A1)** | **Gew.-%** |
|---|---|
| HEMA - Hydroxyethyldimethacrylat | 36,679 |
| Bis-GMA - Bis-phenol-A-glyceryl dimethacrylat | 61,128 |
| BPO - Benzoylperoxid | 2,1 |
| BHT - 2,6 Ditert.-butyl-4-methylphenol 1) | 0,093 |
| | |
| | 100 |
| | |

| **Amin-Harz (B1)** | **Gew.-%** |
|---|---|
| HEMA - Hydroxyethyldimethacrylat | 37,080 |
| Bis-GMA - Bis-phenol-A-glyceryl dimethacrylat | 61,780 |
| BHT - 2,6 Ditert.-butyl-4-methylphenol 1) | 0,01 |
| CQ - Campherchinon | 0,25 |
| DMABSEE - Dimethylaminobenzoesäureethylester 2) | 0,5 |
| DHEPT - N,N-Bis-(2-hydroxyethyl)-p-toluidin | 0,38 |
| | 100 |

| | |
|---|---|
| 1) Stabilisierungsmittel 2) Costarter für den Photoinitiator Campherchinon | |

Mit diesen Komponenten wurde die Haftfestigkeit auf Rinderdentin nach ISO/TS 11405:2003 gemessen.

In einem ersten Schritt wurde das Rinderdentin 15 s mit 35%iger Phosphorsäure geätzt, anschließend wurde mit Wasser gespült.

Anschließend wurde die Elektronendonorlösung (Aktivator) aufgetragen. Im nächsten Schritt wurde der Primer aufgetragen. Die beiden Klebstoffkomponenten A1 und B1 wurden im Volumenverhältnis 1:1 angemischt und auf das mit Primer behandelte Dentin aufgetragen.
Im nächsten Schritt wurde auf diese vorbereitete Oberfläche ein selbsthärtender Füllungskunststoff (Luxacore^{®} der DMG Dental-Material Gesellschaft mbH, 22547 Hamburg) aufgetragen und aushärten gelassen.

Anschließend erfolgte eine Messung der SBS-Haftwerte nach ISO/TS 11405:2003. Der Mittelwert von sieben Messungen war 5,1 ± 1,4 MPa.

### Beispiel 1

Bei diesem Beispiel werden die Elektronendonorverbindung (Aktivator) und als zweite Klebstoffkomponente das Aminharz unverändert aus dem Vergleichsbeispiel 1 übernommen. Als erste Klebstoffkomponente wird eine 1:1 Mischung von Primer und Peroxidharz aus dem Vergleichsbeispiel 1 verwendet.

Bei der Präparation der Prüfkörper und der Messung der SBS-Haftwerte wird wie in Vergleichsbeispiel 1 vorgegangen mit den folgenden Änderungen:
- der Schritt des Auftragens des Primers entfällt,
- auf die geätzte Zahnsubstanz wird unmittelbar eine Mischung von erster Klebstoffkomponente und zweiter Klebstoffkomponente (Aminharz), angemischt im Volumenverhältnis 2:1, aufgetragen.

Es werden neun Messungen des SBS-Haftwertes durchgeführt, der Mittelwert beträgt 8,7 ± 3,3 MPa.

### Beispiel 2

Ein Kit der folgenden Zusammensetzung wird bereitgestellt:

| **Aktivator** | **Gew.-%** |
|---|---|
| Ethanol | 97 |
| Benzolsulfinsäue Na salz | 3 |
| | 100 |
| | |

| **Erste Klebstoffkomponente** | **Gew.-%** |
|---|---|
| wässrige methacrylierte Polyacrylsäure | 10 |
| GDM - Glyceryldimethacrylat | 20 |
| HEMA - Hydroxyethyldimethacrylat | 48,5 |
| Bis-GMA - Bis-phenol-A-glyceryl dimethacrylat | 20 |
| BPO - Benzoylperoxid | 1,5 |
| BHT - 2,6 Ditert.-butyl-4-methylphenol | 0,1 |
| | 100 |
| | |

| **Zweite Klebstoffkomponente** | **Gew.-%** |
|---|---|
| HEMA - Hydroxyethyldimethacrylat | 50 |
| EPDMA - Ethoxyliertes Bis-phenol-A-dimethacrylat | 48,59 |
| BHT - 2,6 Ditert.-butyl-4-methylphenol | 0,01 |
| CQ - Campherchinon | 0,5 |
| DMABSEE - Dimethylaminobenzoesäureethylester | 0,5 |
| DHEPT - N,N-Bis-(2-hydroxyethyl)-p-toluidin | 0,4 |
| | 100 |

Mit diesen Komponenten wurde die Haftfestigkeit auf Rinderdentin nach ISO/TS 11405:2003 gemessen.

In einem ersten Schritt wurde das Rinderdentin 15 s mit 35%iger Phosphorsäure geätzt, anschließend wurde mit Wasser gespült.

Anschließend wurde die Elektronendonorlösung (Aktivator) aufgetragen. Die beiden Klebstoffkomponenten wurden im Volumenverhältnis 1:1 angemischt und auf das geätzte Dentin aufgetragen.

Im nächsten Schritt wurde auf diese vorbereitete Oberfläche ein selbsthärtender Füllungskunststoff (Luxacore^{®} , DMG) aufgetragen und aushärten lassen.

Der SBS-Haftwert als Mittelwert aus acht Messungen betrug 10,6 ± 4,4 MPa.

Die Beispiele 1 und 2 zeigen, dass gegenüber dem Stand der Technik (Vergleichsbeispiel 1) eine erheblich verbesserte Haftung bei gleichzeitig vereinfachter Handhabung erreicht wird.

Die Figuren 1 bis 6 zeigen Applikatoren, in denen ein erfindungsgemäßer Kit bereitgestellt werden kann. Solche Applikatoren für sich genommen sind beschrieben in WO 2007/017225 A2.

Figur 1 zeigt das Grundprinzip eines solchen Applikators. Ein Behälter 1 weist einen Hohlraum 2 auf, in den durch eine erste Öffnung 7 ein Applikatorstift 3 eingesetzt werden kann. Öffnungen 4 erstrecken sich senkrecht zur Achse des Applikatorstiftes 3 und verbinden den Hohlraum 2 mit der Außenwand des Behälters 1. Auf die entsprechenden Öffnungen in der Außenwand des Behälters 1 sind Folienbeutel 5 aufgesetzt. Diese sind Flüssigkeits- und Gasdicht und dienen der Lagerung von erster und zweiter Klebstoffkomponente.

Zur Vorbereitung der Anwendung wird Druck von außen auf die Folienbeutel 5 ausgeübt, so dass diese im Bereich der Öffnungen 4 reißen und der Inhalt in den Hohlraum 2 gedrückt und dort vermischt wird. Das Mischen kann durch den Applikatorstift 3 unterstützt werden. Nach dem Vermischen kann die Mischung mittels des Applikatorstiftes 3 entnommen werden. Die Spitze 11 kann dabei als geeignetes Instrument zum Applizieren der Masse ausgebildet sein, beispielsweise als Pinsel, Schwamm, Spatel oder Kegel.

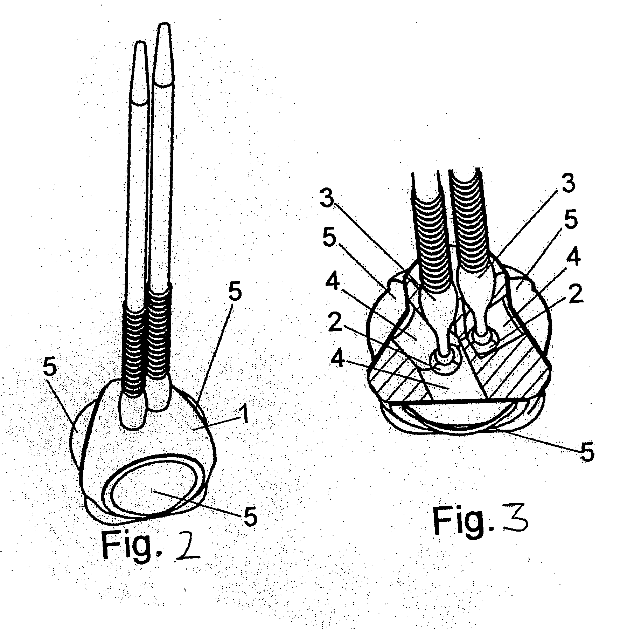

In den Figuren 2 und 3 ist ein Applikator dargestellt, der zwei voneinander getrennte Hohlräume 2 mit jeweils darin eingesetzten Applikatorstiften 3 aufweist. Der in Figuren 2 und 3 linke Hohlraum 2 ist über zwei Öffnungen 4 mit zwei Folienbeuteln 5 verbunden, so dass darin die erste und zweite Klebstoffkomponente angemischt werden können. Der in der Figur rechte Hohlraum ist nur mit einer Öffnung 4 und einem Folienbeutel 5 versehen, es handelt sich hier um die Elektronendonorverbindung.

Bei der Applikation kann zunächst der rechte Folienbeutel 5 zerdrückt und dessen Inhalt in den rechten Behälter 2 gepresst werden. Die Elektronendonorverbindung kann dann mittels des rechten Applikatorstiftes 3 entnommen werden.

Im nächsten Schritt können die beiden linken Folienbeutel mit der ersten und zweiten Klebstoffkomponente zerdrückt und diese beiden Komponenten im linken Hohlraum 2 vermischt werden. Sie können anschließend mittels des linken Applikatorstiftes 3 entnommen und auf die Zahnsubstanz aufgetragen werden.

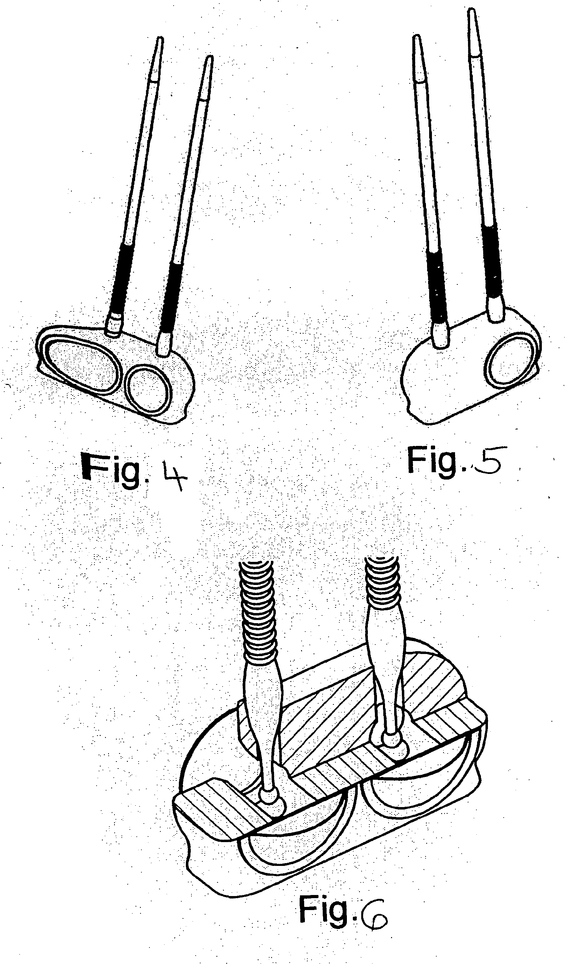

Die Figuren 4 bis 6 zeigen eine weitere Ausführungsform eines erfindungsgemäßen Applikators. Der in Figur 4 linke Hohlraum ist mit zwei Öffnungen und entsprechenden Folienbeuteln versehen, deren Inhalt im zugehörigen Hohlraum 2 gemischt werden kann. Diese beiden Folienbeutel enthalten die erste und zweite Klebstoffkomponente. Der in Figur 4 rechte Hohlraum ist mit nur einem Folienbeutel verbunden, der die Elektronendonorverbindung enthält.

## Patentansprüche

1. Kit zum Befestigen eines dentalen Restaurationsmaterials an einer Zahnsubstanz, **gekennzeichnet durch** folgende Komponenten:
a. eine Elektronendonorverbindung;
b. eine erste Klebstoffkomponente, die ein Oxidationsmittel als Bestandteil eines Polymerisationsinitiatorsystems und eine polymerisierbare Säure enthält;
c. eine zweite Klebstoffkomponente, die ein Reduktionsmittel als Bestandteil eines Polymerisationsinitiatorsystems und ein polymerisierbares Monomer, Oligomer oder Präpolymer enthält, das keine Säuregruppen enthält.

2. Kit nach Anspruch 1, **dadurch gekennzeichnet, dass** die Elektronendonorverbindung in einem physiologisch akzeptablen Lösungsmittel vorliegt.

3. Kit nach Anspruch 2, **dadurch gekennzeichnet, dass** das physiologisch akzeptable Lösungsmittel bei Körpertemperatur leichtflüchtig ist.

4. Kit nach Anspruch 3, **dadurch gekennzeichnet, dass** das physiologisch akzeptable Lösungsmittel ein Alkohol, vorzugsweise Ethanol, ist.

5. Kit nach einem der Ansprüche 1 bis 4, **dadurch gekennzeichnet, dass** das Reduktionsmittel ein Amin enthält.

6. Kit nach Anspruch 5, **dadurch gekennzeichnet, dass** das Amin ein tertiäres aromatisches Amin ist.

7. Kit nach einem der Ansprüche 1 bis 6, **dadurch gekennzeichnet, dass** die polymerisierbare Säure polymerisierbare Homo- und/oder Copolymere einer α,β-ungesättigten Carbonsäure umfasst.

8. Kit nach einem der Ansprüche 1 bis 7, **dadurch gekennzeichnet, dass** die polymerisierbare Säure eine mittlere Molmasse von 2.000 bis 100.000, vorzugsweise 20.000 bis 40.000 aufweist.

9. Kit nach einem der Ansprüche 1 bis 8, **dadurch gekennzeichnet, dass** das Oxidationsmittel ein organisches Peroxid ist.

10. Kit nach einem der Ansprüche 1 bis 9, **dadurch gekennzeichnet, dass** das säuregruppenfreie polymerisierbare Monomer ein vinylgruppenhaltiges Monomer, vorzugsweise ein Acrylat oder Methacrylat, umfasst.

11. Kit nach einem der Ansprüche 1 bis 10, **dadurch gekennzeichnet, dass** die erste und/oder zweite Klebstoffkomponente zusätzlich einen Filmbildner aufweist.

12. Kit nach Anspruch 11, **dadurch gekennzeichnet, dass** der Filmbildner ausgewählt ist aus der Gruppe bestehend aus 2-Hydroxymethylmethacrylat; 2- und 3-Hydroxypropylacrylat; 2- und 3-Hydroxypropylmethacrylat; 1,3- und 2,3-Dihydroxypropylacrylat; und 1,3- und 2,3-Dihydroxypropylmethacrylat.

13. Kit nach Anspruch 11 oder 12, **dadurch gekennzeichnet, dass** der Anteil des Filmbildners an der Gesamtmasse der ersten und zweiten Klebstoffkomponente mindestens 30 Gew.-%, vorzugsweise mindestens 40 Gew.-%, weiter vorzugsweise mindestens 50 Gew.-% beträgt. Bevorzugte Obergrenzen sind 70 Gew.-%, besonders bevorzugt 60 Gew.-%.

14. Kit nach einem der Ansprüche 1 bis 13, **dadurch gekennzeichnet, dass** die erste und zweite Klebstoffkomponente gemeinsam in einem Applikator bereitgestellt werden, in dem sie getrennt gelagert sind, und der Einrichtungen zur Vermischung der ersten und zweiten Klebstoffkomponente und zur Entnahme der Mischung aufweist.

15. Kit nach Anspruch 14, **dadurch gekennzeichnet, dass** der Applikator zusätzlich die Elektronendonorverbindung bereitstellt, die getrennt von den Klebstoffkomponenten gelagert und separat entnehmbar ist.

16. Kit nach einem der Ansprüche 1 bis 15, **dadurch gekennzeichnet, dass** er zusätzlich eine Säurekomponente zum Ätzen der Zahnsubstanz enthält.

17. Kit nach einem der Ansprüche 1 bis 16, **dadurch gekennzeichnet, dass** er zusätzlich ein dentales Restaurationsmaterial enthält.

18. Kit nach einem der Ansprüche 1 bis 17, **dadurch gekennzeichnet, dass** er zusätzlich eine Anleitung zur Applikation umfasst.

19. Kit nach Anspruch 14, **dadurch gekennzeichnet, dass** die Anleitung zur Applikation folgende Schritte umfasst:
a. Ätzen der Zahnsubstanz mit einer Säure,
b. Auftragen der Elektronendonorverbindung auf die geätzte Zahnsubstanz,
c. Mischen der ersten und zweiten Klebstoffkomponente und Auftragen der Mischung auf die Zahnsubstanz,
d. Applizieren des dentalen Restaurationsmaterials auf die Zahnsubstanz.

20. Verwendung der folgenden Komponenten:
a. eine Elektronendonorverbindung;
b. eine erste Klebstoffkomponente, die ein Oxidationsmittel als Bestandteil eines Polymerisationsinitiatorsystems und eine polymerisierbare Säure enthält;
c. eine zweite Klebstoffkomponente, die ein Reduktionsmittel als Bestandteil eines Polymerisationsinitiatorsystems und ein polymerisierbares Monomer, Oligomer oder Präpolymer enthält, das keine Säuregruppen enthält;
zur Herstellung eines Kits zum Befestigen eines dentalen Restaurationsmaterials an einer Zahnsubstanz, wobei die Komponenten des Kits hergerichtet sind zur Applikation umfassend folgende Schritte:
- Ätzen der Zahnsubstanz mit einer Säure,
- Auftragen der Elektronendonorverbindung auf die geätzte Zahnsubstanz,
- Mischen der ersten und zweiten Klebstoffkomponente und Auftragen der Mischung auf die Zahnsubstanz,
- Applizieren des dentalen Restaurationsmaterials auf die Zahnsubstanz.
